# EUROPEAN PATENT SPECIFICATION

(11) **EP 1 874 921 B1**
(45) Date of publication and mention of the grant of the patent: **22.06.2011**
(21) Application number: 06768467.0
(22) Date of filing: 21.04.2006
(51) Int. Cl.: C12N 5/0775

(54) **TRANSPLANTATION OF DIFFERENTIATED IMMATURE ADIPOCYTES AND BIODEGRADABLE SCAFFOLD FOR TISSUE AUGMENTATION**
TRANSPLANTATION DIFFERENZIERTER UNREIFER ADIPOCYTEN UND BIOLOGISCH ABBAUBARES GERÜST ZUR GEWEBEVERMEHRUNG
TRANSPLANTATION D'ADIPOCYTES IMMATURES DIFFERENCIÉS ET SQUELETTE BIODÉGRADABLE D'AUGMENTATION TISSULAIRE

(30) Priority: 21.04.2005 KR 20050033091
(43) Date of publication of application: 09.01.2008
(73) Proprietor: Anterogen Co., Ltd., Seoul 156-811 (KR)
(72) Inventor: LEE, Sung-Koo, Seocho-gu, Seoul 137-030 (KR); KIM, Mi-Hyung, Kangnam-gu, Seoul 135-110 (KR); KIM, In-Ok, Seoul 150-057 (KR)
(74) Representative: Pohlman, Sandra M.
(86) International application number: PCT/KR2006/001516
(87) International publication number: WO 2006/112684

(56) References cited:
- WO-A-00/44882
- WO-A2-03/053346
- US-A- 6 153 432
- TCHOUKALOVA YOURKA D ET AL: "A quick, reliable, and automated method for fat cell sizing." JOURNAL OF LIPID RESEARCH, vol. 44, no. 9, September 2003 (2003-09), pages 1795-1801, XP002510268 ISSN: 0022-2275
- KIMURA Y ET AL: "Adipose tissue engineering based on human preadipocytes combined with gelatin microspheres containing basic fibroblast growth factor" BIOMATERIALS, ELSEVIER SCIENCE PUBLISHERS BV., BARKING, GB, vol. 24, no. 14, 1 June 2003 (2003-06-01), pages 2513-2521, XP004419996 ISSN: 0142-9612
- VON HEIMBURG D ET AL: "Human preadipocytes seeded on freeze-dried collagen scaffolds investigated in vitro and in vivo" BIOMATERIALS, ELSEVIER SCIENCE PUBLISHERS BV., BARKING, GB, vol. 22, no. 5, 1 March 2001 (2001-03-01), pages 429-438, XP004227888 ISSN: 0142-9612
- VON HEIMBURG D. ET AL.: 'Human preadipocytes seeded on freeze-dried collagen scaffolds investigated in vitro and in vivo' BIOMATERIALS vol. 22, 2001, pages 429 - 438, XP004227888
- HALBLEIB M. ET AL.: 'Tissue engineering of white adipose tissue using hyaluronic acid-based scaffolds. I: in vitro differentiation of human adipocyte precursor cells on scaffolds' BIOMATERIALS vol. 24, 2003, pages 3125 - 3132, XP004441793
- KIMURA Y. ET AL.: 'Adipose tissue engineering based on human preadipocytes combined with gelatin microspheres containing basic fibroblast growth factor' BIOMATERIALS vol. 24, 2003, pages 2513 - 2521, XP004419996
- TORIYAMA K. ET AL.: 'Endogenous adipocyte precursor cells for regenerative soft-tissue engineering' TISSUE ENG. vol. 8, February 2002, pages 157 - 165, XP008123805

## Description

### Technical Field

The present invention relates to a technology involving differentiating preadipocytes, derived from human adipose tissues, into adipocytes and transplanting the differentiated adipocytes in conjunction with a biodegradable scaffold into the body. More specifically, the present invention relates to immature adipocytes having a cell diameter of 20 to 40 µm, which are obtained by isolating preadipocytes from autologous or allogeneic human adipose tissues and culturing the isolated preadipocytes in a medium containing growth factors, followed by differentiation adipocytes, a method of culturing the immature adipocytes with a biodegradable scaffold, and a method of transplanting the immature adipocytes in conjunction with a biodegradable scaffold into the body.

### Background Art

At present, autologous transplantation of fat is prevalently performed. History of autologous fat transplantation dates back to 1890's and this technique has been widely used with the introduction of a liposuction technique in 1980's. As a result, there are accumulation of many cases and documents with verified evidence showing safety of autologous fat transplantation. In particular, fat transplantation is widely practiced for various applications such as alleviation or reduction of wrinkles of aged skin, correction and reconstruction of lip line, face line and hairline, and rhinoplasty. In addition, fat transplantation is conducted in regions of depressed skin resulting from skin bums or wounds, tissue defects created by carcinectomy and the like, with some satisfactory results as a corrective surgery of the defect area.

However, transplantation of fat, which was obtained from fat tissues by simple liposuction, showed disappointing outcomes with short-term survival and duration of fat transplants including a high absorption rate of 40 to 60% in the volume of transplanted fat after surgery and therefore re-transplantation of fat is required (S Eremia et al., 2000, Dermatol. Sur. 26, 1150-1158; and Fulton JE et al., 2001, Dermatol. Clin. 19(3), 523-530).

For these reasons, a great deal of study has been continued to isolate pure adipocytes from bulk fat obtained by liposuction. As an effort to achieve this purpose, some research groups and institutions have conducted fat transplantation after removal of fibrous materials and blood from the liposuctioned bulk fat, but there is still no satisfactory result hitherto (G Sattler et al., 2000, Dermatol. Sur. 26, 1140-1144).

As discussed above, when fat simply isolated from adipose tissues are transplanted into the body, reduction of the transplant volume occurs. This is because most of adipocytes thus obtained are those which are fully matured, and considerable portions of cells are destroyed during liposuction since mature adipocytes are fragile by pressure, and as a result, an engraft rate thereof is lowered after transplantation and revascularization or neovascularization does not progress favorably.

To this end, a great deal of research has been and is being actively undertaken for the potential applications of preadipocytes contained in adipose tissues as an alternative of fat transplantation. As adipose tissue-derived preadipocytes may differentiate into osteocytes, adipocytes, myocytes, neurocytes and the like, depending upon given differentiation conditions, such an approach is based on the application of a mechanism involving in vivo transplantation of preadipocytes and differentiation thereof into adipocytes after being engrafted into the target site (JM Gimble et al., 2000, Bone 19: 421-428). However, preadipocytes exhibit multipotency as they are, and therefore safe transplantation is not secured. In addition, differentiation potential and differentiation rate of preadipocytes into adipocytes are significantly affected by physiological microenvironment surrounding the target transplantation site and therefore it is difficult to accurately predict the results which may occur after transplantation. Further, combined treatment of growth factors as a supportive therapy to help preadipocytes to be differentiated into adipocytes may cause safety problems.

To cope with such problems, US Patent No. 6,153,432 (issued on Nov. 28, 2000) discloses a method of isolating undifferentiated preadipocytes from adipose tissues obtained via liposuction surgery and differentiating the isolated preadipocytes into adipocytes necessary for transplantation, and reagents for the same. Specifically, this method involves isolating preadipocytes from adipose tissues obtained via liposuction; suspending the isolated preadipocytes in a stromal medium, inoculating the suspended cells into a culture vessel, and incubating them in a CO₂ incubator at 37°C for 24 hours until preadipocytes attach on the bottom of the culture vessel; replacing the stromal medium with a differentiation medium and incubating the cells for 3 days; and replacing the differentiation medium with an adipocyte medium and differentiating preadipocytes into adipocytes. According to this method, it is possible to maximize desired effects by transplantation of healthy and immature adipocytes only, instead of using aged adipocytes which are typically used in conventional fat transplantation techniques, and it is also possible to overcome problems associated with transplantation safety and effectiveness which may arise from the use of preadipocytes.

However, the method disclosed in US Patent No. 6,153,432 uses only the expression "differentiated adipocytes containing lipid droplets (LDs)" without specific definition of differentiated adipocytes, therefore suffers from the problems of difficulty to definitely specify and confirm the optimal harvest period of differentiated adipocytes which will be finally obtained from preadipocytes and the quality of the resulting adipocytes.

Preadipocytes mature through the following differentiation stages: formation of small lipid droplets in the cytoplasm, at an initial stage of differentiation, a gradual increase of small lipid droplets in number thereof, and aggregation of small lipid droplets into large lipid droplets. Once fully matured, one lipid droplet becomes to occupy the entire cytoplasm and a size of the cell also gradually increases with lipid accumulation during the maturation process. Preadipocytes, as they cannot ensure their differentiation into adipocytes after transplantation, and the fully-matured adipocytes may be destroyed during a cell harvesting process or may exhibit lowering of an engraft rate after in vivo transplantation thereof. Consequently, there is a need in the related art for the development of a method capable of maximizing desired transplantation effects by specifically defining characteristics of cells for use in transplantation and then securing healthy and immature adipocytes.

In the last several years, there has been remarkable growth in the biomaterial field. Large numbers of materials have already entered clinical application and for example, collagen among them is the representative material. In addition, various kinds of biomaterials are used in the tissue engineering field and include for example, poly-lactic acid, poly-glycolic acid, collagen type I derivatives and alginate. It may also be possible to facilitate the growth of cells within the transplanted matrix by transplantation of such biomaterials in conjunction with treatment of exogenous factors such as steroids or growth hormones. According to recently published articles, it was confirmed that co-injection of bFGF (basic fibroblast growth, factor) and Matrigel (basement membrane collagen) into mice results in formation of new adipose tissues (K Toriyama et al., 2002, Tissue Engineering, vol. 8, 157-165). That is, it was confirmed that endothelial cells gather around Matrigel, resulting in formation of new blood vessels (neovascularization) and lipid droplets are formed into the thus-gathered fibroblast-like cells. In addition, when preadipocytes are introduced into a polymer scaffold made of poly lactic-co-glycolic acid (PLGA) which is then transplanted into rats, preadipocytes differentiate into adipocytes, thereby forming adipose tissues (CW Patrick et al., 2002, Tissue Engineering, vol. 8, 283-293).

Despite remarkable advancement in the biomaterial field, there is still no development of a method capable of effectively regenerating adipose tissues via transplantation of the biomaterial into the human. The most important reason for this is no development of optimal conditions capable of effectively proliferating and differentiating human autologous or allogeneic adipocytes, and conditions for obtaining an appropriate form of differentiated adipocytes which can be engrafted at a high rate and maintained for a long period of time upon transplantation. As a consequence, in order to effectively treat deficiencies or defects of soft tissues, there is a need for intimate harmony between the cell production technology and the relevant biomaterial.

### Disclosure

### Technical Problem

Therefore, the present invention has been made in view of the above problems, and it is an object of the present invention to provide a method capable of effectively replacing the body volume of the target site via formation of adipose tissues like naturally-occurring adipocytes upon in vivo transplantation in conjunction with a biodegradable scaffold, by establishment of a technique for producing implantable differentiated immature adipocytes using undifferentiated preadipocytes isolated from human adipose tissues and confirmation of maturation of adipocytes within the biodegradable scaffold following in vitro co-culturing of the isolated preadipocytes with the biodegradable scaffold.

### Technical Solution

In accordance with an aspect of the present invention, the above and other objects can be accomplished by the provision of an implantable adipocyte composition for replacing a body volume, comprising immature adipocytes having a cell diameter of 20 to 40 µm, which are obtained by differentiation of adipose tissue-derived preadipocytes into adipocytes.

In accordance with another aspect of the present invention, there is provided a method for producing an implantable adipocyte composition, comprising:
(a) differentiating undifferentiated preadipocytes, isolated from autologous or allogeneic adipose tissues, into adipocytes;
(b) collecting differentiated immature adipocytes having a cell diameter of 20 to 40 µm; and
(c) culturing the collected immature adipocytes with a biodegradable scaffold in vitro.

In accordance with yet another aspect of the invention, there is provided an implantable adipocyte composition, obtained according to the aforementioned method of the invention, for use in transplanting adipocytes, wherein the composition comprises immature adipocytes in conjunction with the biodegradable scaffold to be transplanted into a body.
Also described herein is a method for transplanting adipocytes, comprising:
(a) differentiating undifferentiated preadipocytes, isolated from autologous or allogeneic adipose tissues, into adipocytes;
(b) collecting differentiated immature adipocytes having a cell diameter of 20 to 40 µm;
(c) culturing the collected immature adipocytes with a biodegradable scaffold in vitro; and
(d) transplanting the immature adipocytes in conjunction with the biodegradable scaffold into a body.

In differentiation of undifferentiated preadipocytes, isolated from autologous or allogeneic adipose tissues, into adipocytes, the present invention is characterized in that adipocytes having a maximized differentiation rate are obtained by culturing preadipocytes using appropriate stromal medium, expansion medium, differentiation medium and maintenance medium and collecting immature adipocytes matured to a predetermined size.

Adipocytes utilized in the present invention for transplantation are differentiated immature adipocytes having a cell diameter of 20 to 40 µm, and mature at the target transplantation site with a gradual increase in volume thereof. Immature adipocytes at this stage provide advantages such as no need for differentiation-inducing factors unlike preadipocytes, superior survival rate and engraft rate as compared to mature adipocytes or adipocytes obtained by any other methods, and gradual volume increases at transplantation site.

When immature adipocytes obtained in the present invention are transplanted in combination with a scaffold, the volume replacement at an early stage of transplantation is effected by the scaffold which is biodegraded and absorbed during engrafting and growth of adipocytes, and then the defect area for transplantation is replaced with adipose tissues having the same morphology and characteristics as the native adipose tissues. Therefore, the implantable adipocyte composition of the present invention results in maturation of adipocytes with a gradual increase of the volume at the target transplantation site and thereby can be used as an effective replacement of the body volume, and can treat disorders due to defects of soft tissues or aesthetic defects in appearance.

### Description of the Drawings

The above and other objects, features and other advantages of the present invention will be more clearly understood from the following detailed description taken in conjunction with the accompanying drawings, in which:
FIG. 1 is a micrograph (X 400) showing a size of differentiated immature adipocytes and mature adipocytes as determined by Oil Red O staining;
FIG. 2 is a micrograph (A, C-F: X 400) of mature adipocytes stained with Oil Red O, after introduction of differentiated immature adipocytes into fibrin and co-culturing of them for one month;
FIG. 3 is a graph showing changes in amounts of leptin secreted into the culture supernatant, with introduction of differentiated immature adipocytes into fibrin and co-culturing of them; and
FIG. 4 is a micrograph (A: X 40 and B-D: X 400) of adipocytes stained with Oil Red O, after introduction of differentiated immature adipocytes into alginate bead and co-culturing of them.

### Best Mode

Hereinafter, the present invention will be described in more detail.

As used herein, the term "preadipocyte" refers to a cell isolated from adipose tissues, particularly a cell having a potency of differentiation into adipocyte.

As used herein, the term "adipocyte" is a cell which forms lipid droplets and secretes leptin via treatment of a preadipocyte with a differentiation-inducing factor, and refers to an adipocyte differentiated from an undifferentiated preadipocyte, in the present invention.

As used herein, the term "differentiated immature adipocyte" is a cell which forms lipid droplets and secretes leptin via treatment of a preadipocyte with a differentiation-inducing factor, and refers to an adipocyte which has a cell diameter of 20 to 40 µm and is differentiated from an undifferentiated preadipocyte, in the present invention.

As used herein, the term "preadipocyte-differentiation factor" refers to a substance necessary for differentiation of preadipocytes into adipocytes, and includes for example, biotin, insulin, pantothenate, dexamethasone, isobutylmethylxantine (IBMX) and indomethacin.

As used herein, the phrase "transplantation for clinical application" refers to transplantation of immature adipocytes differentiated from preadipocytes, in order to correct physical contours such as alleviation or reduction of skin wrinkles due to senescence or correction of face contours, or in order to regenerate depressed areas throughout the body, such as tissue defects due to carcinectomy, depressed regions due to cut wound, depressed areas due to physical deformity and the like.

As used herein, the term "allogeneic transplantation" is grafting of specific tissues or organs or cells derived from another person or other animals belonging to the same specifies, and refers to transplantation of tissues or organs or cells derived from other people or other animals when it is impossible to use autologous tissues or organs or cells.

The present invention includes differentiation of undifferentiated preadipocytes, isolated from autologous or allogeneic adipose tissues, into adipocytes, and various methods for differentiation of preadipocytes are already published in a large amount of literature. The present invention proposes a method for producing a cell which is capable of achieving a gradual volume increase after transplantation, via improvement of the method disclosed in the above-cited US Patent No. 6,153,432 (issued on Nov. 28, 2000).

Preadipocytes that can be utilized in the present invention are primarily obtained from human subcutaneous adipose tissues via liposuction without being limited thereto.

Differentiated adipocytes of the autologous or allogeneic origin, for use in transplantation in accordance with the present invention, may be obtained according to the following procedure:

### (1) Isolation of preadipocytes from adipose tissues obtained via liposuction

Adipose tissues are washed with KRB solution (Krebs-Ringer bicarbonate solution), treated with collagenase and then centrifuged to remove a top fat layer, and a stromal medium is suspended in the bottom layer, followed by centrifugation to recover preadipocytes from the bottom layer.

### (2) Culture of preadipocytes in stromal medium

Preadipocytes are suspended in the stromal medium, inoculated into a culture vessel and cultured until cells attach on the bottom of the culture vessel. Cells are cultured for 24 hours using DMEM/F12 (Dulbecco's Modified Eagle Medium/Ham's F-12 Nutrient Broth) containing 10% fetal bovine serum (FBS) as the stromal medium.

### (3) Cell culture in expansion medium

After removing the stromal medium, cells are cultured in the expansion medium such that sufficient amounts of preadipocytes are expanded. The expansion medium used herein is DMEM/F12 containing 10% FBS, EGF (epidermal growth factor), bFGF (basic fibroblast growth factor) and TGF-beta 1 (transforming growth factor beta 1) which serves to increase cell amounts to large quantities by inducing rapid proliferation of preadipocytes.

### (4) Cell culture in stromal medium

When the bottom of the culture vessel is full of cells as a monolayer, the expansion medium is replaced with a stromal medium and cells are additionally cultured for 1 to 3 days.

### (5) Cell culture in differentiation medium

The stromal medium is removed and cells are cultured in a differentiation medium. The differentiation medium used herein is DMEM/F12 containing 3% FBS, biotin, pantothenate, insulin, dexamethasone, isobutylmethylxanthine (IBMX) and indomethacin, and serves to induce differentiation of preadipocytes into adipocytes, thereby initiating formation of small lipid droplets in the cytoplasm.

### (6) Cell culture in maintenance medium

The differentiation medium is removed and cells are cultured in a maintenance medium. The maintenance medium used herein is a modified form of the differentiation medium from which cell differentiation-inducing isobutylmethylxanthine (IBMX) and indomethacin were excluded, and is replaced with a fresh medium every 2 or 3 days. Upon culturing in the maintenance medium, cells mature into adipocytes with increasing numbers of lipid droplets therein and gradually increasing cell sizes.

In particular, when preadipocytes are isolated from initial adipose tissues and aliquoted into the culture vessel, the cell is adjusted to a fat concentration of about 0.04 mℓ/cm² for liposuctioned fat and cultured for 6 to 8 days to ensure sufficient proliferation. Without subculturing, cell multiplication is terminated at passage 0 (P 0), followed by differentiation to obtain adipocytes having a maximized differentiation rate.

Harvesting period of adipocytes thus differentiated may be determined as follows. That is, preadipocytes begin to form lipid droplets in the differentiation medium. With an increasing incubation time in the maintenance medium, numbers and size of lipid droplets are increased, which results in matured cells. Once sufficiently matured, lipid droplets combine into one which becomes to occupy the entire cytoplasm by accumulating additional lipid. During culturing process, cells are detached from the bottom of the culture vessel, thereby becoming afloat. In addition, preadipocytes gradually increase in size as they undergo differentiation stages, and the diameter of adipocytes at the early stage of differentiation is in the range of about 10 to 20 µm and the size of fully-matured adipocytes is in the range of about 70 to 120 µm. According to the present invention, it is preferred to harvest cells when differentiated adipocytes reached the cell diameter of 20 to 40 µm.

According to the present invention, differentiated immature adipocytes and a biodegradable scaffold are co-cultured in vitro, and binding and maturation of adipocytes within the biodegradable scaffold are determined as follows.

### (1) Harvesting of differentiated immature adipocytes

Adipocytes cultured in a maintenance medium are maintained with exchange of the culture medium with a fresh medium at an interval of 2 to 3 days. Adipocytes cultured for 6 to 9 days are collected using trypsin/EDTA and a cell size is determined. Particularly, when the thus-collected cells are applied to the human, it is preferred that culture media prior to 2 to 4 days of cell collection does not contain bovine sera.

### (2) Biodegradable scaffold

Materials for the biodegradable scaffold may include, but are not limited to, fibrin, alginate, PLGA (poly lactic-co-glycolic acid) and PTFT (polytetrafluoroethylene). Forms of the biodegradable scaffold also include both injectable ones via injection and implantable ones as structures via a surgical operation.

### (3) Transplantation of differentiated immature adipocytes into biodegradable scaffold

Immature adipocytes having a cell diameter of 20 to 40 µm are transplanted into the biodegradable scaffold and cultured in a culture medium.

### (4) Determination of binding and maturation of adipocytes within biodegradable scaffold

Engrafting and maturation of adipocytes within the biodegradable scaffold are determined by the following method:
① A method involving identifying adipocytes by Oil Red O staining and determining maturation and size thereof; or
② A method of confirming functions of adipocytes by quantifying leptin in the cell culture supernatant.

When immature adipocytes obtained as above were transplanted into the biodegradable scaffold, it was confirmed that the transplanted cells well engrafted on the target site and fully matured with increasing lipid droplet size and subsequently cell size. In addition, secretion of leptin, which is the characteristic function of the adipocyte, was also continuously increased.

Further, the present invention includes the step of co-transplanting the differentiated immature adipocytes and biodegradable scaffold into the body. That is, autologous or allogeneic adipocytes, obtained according to the method of the present invention, can be used in transplantation for clinical application, in conjunction with the biodegradable scaffold.

### Mode for Invention

Now, the present invention will be described in more detail with reference to the following examples. These examples are provided only for illustrating the present invention and should not be construed as limiting the scope and spirit of the present invention.

### Example 1: Production of immature adipocytes

Firstly, preadipocytes were isolated from adipose tissues obtained via liposuction as follows: in order to remove blood, the adipose tissues were washed 3 or 4 times with the same volume of a KRB solution. The same volume of a collagenase solution as that of the adipose tissues was added thereto and the materials were reacted in a water bath at 37°C. The resulting reaction solution was transferred to a centrifugal tube and centrifuged at 1200 rpm and 20°C for 10 minutes. The lipid and fat layer as the supernatant were removed, and the lower layer, i.e. a collagenase solution was carefully separated without being shaken. A stromal medium was suspended therein, followed by centrifugation at 1200 rpm and 20°C for 5 minutes. Here, preadipocytes were allowed to settle and the supernatant were removed.

The thus-obtained preadipocytes were suspended in the stromal medium, inoculated into a culture vessel, and cultured in a 5% CO₂ incubator at 37°C for 24 hours such that cells adhered to the bottom. The stromal medium used herein is DMEM/F12 (Dulbecco's Modified Eagle Medium/Ham's F-12 Nutrient Broth) containing 10% fetal bovine serum.

Then, the culture medium was periodically replaced with an expansion medium and cells were continuously cultured until the bottom of the culture vessel was full of cells as a monolayer. The composition of the expansion medium is as follows: DMEM/F12, 10% FBS, 5 ng/mℓ EGF, 0.25 ng/mℓ bFGF, and 0.25 ng/mℓ TGF-b1.

When the bottom of the culture vessel was full of cells as a monolayer, the expansion medium was replaced with a stromal medium and cells were cultured for additional 3 days.

Then, the stromal medium was replaced with a differentiation medium and cells were cultured for 3 days. The composition of the differentiation medium is as follows: DMEM/F12, 3% FBS, 33 µM biotin, 17 µM pantothenate, 1 µM insulin, 1 µM dexamethasone, 250 µM isobutylmethylxanthine (IBMX) and 100 µM indomethacin.

Next, the differentiation medium was replaced with an adipocyte medium (maintenance medium) and cells were cultured until the diameter of the collected cells reached 20 to 40 µm as lipid droplets grew. The composition of the adipocyte medium is as follows: DMEM/F12, 3% FBS, 33 µM biotin, 17 µM pantothenate, 100 µM insulin and 1 µM dexamethasone.

### Example 2: Engrafting of immature adipocytes into scaffold and Co-culture thereof

### (1) Harvesting of differentiated immature adipocytes

A trypsin/EDTA solution was added to a flask containing immature adipocytes which were produced in Example 1. The resulting mixture was reacted in an incubator for 1 to 15 minutes and DMEM/F12 was added thereto, thereby inactivating the trypsin/EDTA solution.

The solution was collected and then centrifuged at 1200 rpm and 20°C for 5 minutes. The supernatant was removed and a shipping medium (phenol red-free DMEM) was added to suspend the cells, and the mixture was centrifuged once again under the same conditions as above. The supernatant was removed and an appropriate amount of the shipping medium was added, followed by cell counting. The amount of the shipping medium which will be finally added was calculated and centrifugation was repeated again.

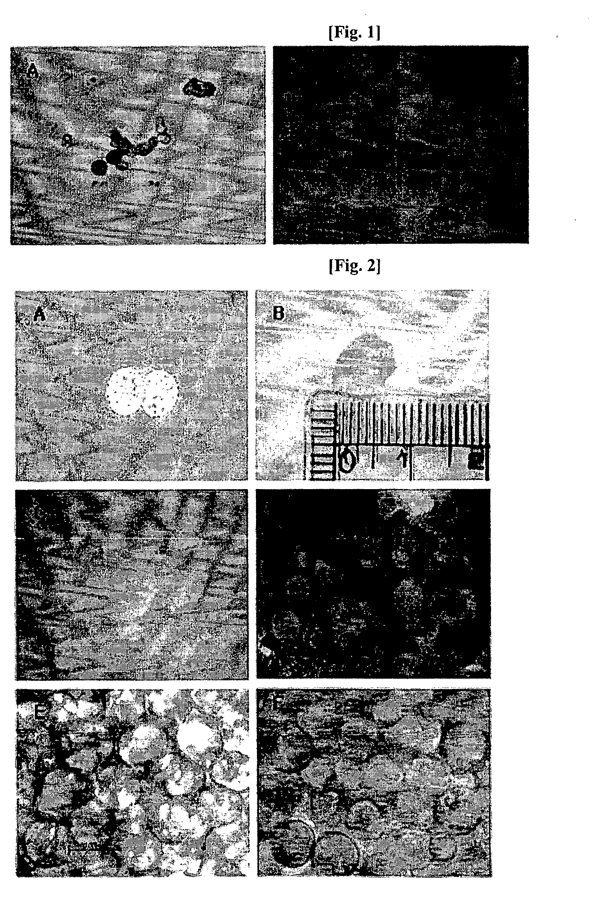

FIG. 1 is a micrograph (X 400) showing a size of differentiated immature adipocytes and mature adipocytes as measured by Oil Red O staining. FIG. 1A shows a cell size measured after collection of differentiated immature adipocytes, wherein the cells contain several numbers of lipid droplets and have a cell diameter of 20 to 40 µm. FIG. 1B is a micrograph of mature adipocytes stained with Oil Red O, after three-dimensional culture of the collected immature adipocytes in vitro, wherein several lipid droplets merge into one large one and the cells have a cell diameter of 60 to 100 µm.

### (2) Co-culture of differentiated immature adipocytes and fibrin

A fibrinogen solution and a thrombin solution were prepared 10 minutes prior to use, as follows: an aprotinin solution was placed in a vial containing lyophilized fibrinogen, which was then allowed to stand for 1 to 2 minutes and gently shaken to be completely dissolved. The thrombin solution was prepared by placing a calcium chloride solution in a vial containing thrombin and shaking the vial until the contents were completely dissolved.

The thrombin solution was added to immature adipocytes obtained by removing the supernatant after final centrifugation in Step (1), such that cells were suspended to a concentration of 1 to 5 x 10⁷ cells/mℓ. 100 µℓ of the thrombin-cell suspension and 100 µℓ of the fibrinogen solution were homogeneously mixed. When a fibrin gel to which immature adipocytes were introduced is formed 1 to 2 minutes later, the adipocyte medium was added and co-cultured with the periodic replacement of the culture medium at a 3-day interval.

FIG. 2 is a micrograph(A, C-F: X 400) of mature adipocytes stained with Oil Red O, after introduction of differentiated immature adipocytes into fibrin and co-culturing of them for one month. FIG. 2A is a micrograph of differentiated immature adipocytes after collection thereof, wherein the cells contain several lipid droplets. FIG. 2B is a micrograph of fibrin-cells co-cultured for 1 month after introduction of the collected immature adipocytes into fibrin. FIGs. 2C and 2D are respectively micrographs of adipocytes matured within the fibrin gel and mature adipocytes stained with Oil Red O, showing that they have a size and morphology similar to human adipose tissues (FIGs. 2E and 2F) stained with Oil Red O.

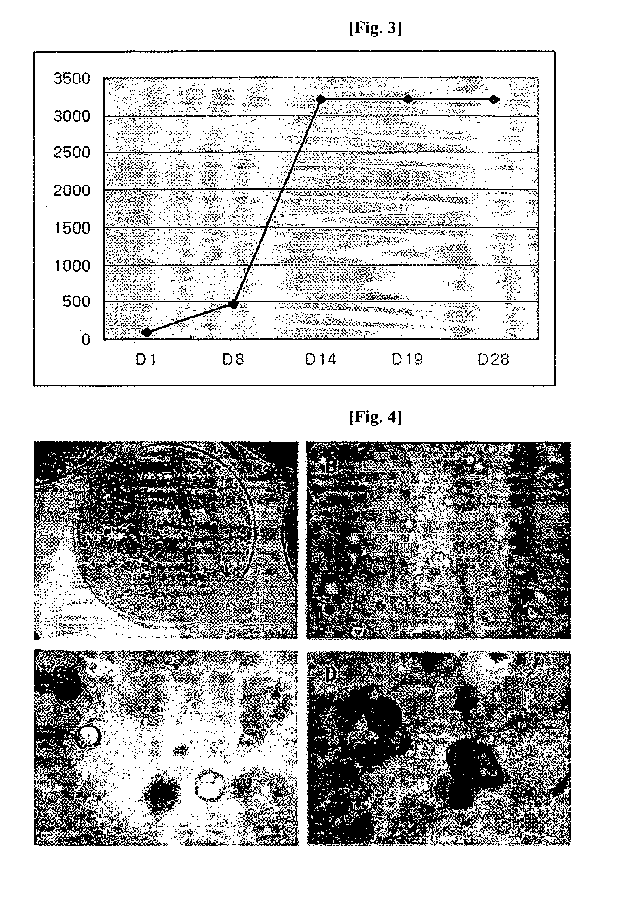

FIG. 3 is a graph showing changes in amounts of leptin secreted into the culture supernatant, with introduction of differentiated immature adipocytes into fibrin and co-culturing of them. As can be seen therefrom, co-culturing of fibrin-cells resulted in continuous increases of leptin secretion from Day 1 to Day 14 of co-culture, with a steady state after Day 14 of culture.

As can be seen from FIGs. 2 and 3, differentiated immature adipocytes fully mature via three-dimensional culture within the fibrin gel, and therefore show cytobiological similarity with naturally-occurring adipose tissues formed in the body. Fibrin is non-toxic to immature adipocytes, and is decomposed during the maturation of immature adipocytes, thereby providing spaces for growth in size of adipocytes. As a result, it is possible to maximize transplantation effects of immature adipocytes.

### (3) Co-culture of differentiated immature adipocytes and alginate beads

Alginate was dissolved in PBS to make a 2% solution which was then sterilized and stored at room temperature. A 102 mM calcium chloride solution and a 150 mM sodium chloride solution were respectively prepared, sterilized and stored at room temperature.

The alginate solution was added to immature adipocytes obtained by removing the supernatant after final centrifugation in Step (1), such that cells were suspended to a concentration of 1 to 5 x 10⁷ cells/mℓ. The alginate-cell suspension was placed in a syringe, added dropwise to the calcium chloride solution, gently shaken and allowed to stand for 10 min. When alginate beads (a diameter of about 1 mm) containing immature adipocytes were formed, the calcium chloride solution was removed and the thus-formed beads were washed four times with the sodium chloride solution. After removing the sodium chloride solution, an adipocyte medium was added to the beads and the mixture was co-cultured with the replacement of the culture medium at a 3-day interval.

FIG. 4 is a micrograph (A: X40, and B-D: X400) of adipocytes stained with Oil Red O, after introduction of differentiated immature adipocytes into alginate beads and co-culturing of them. 4A: micrograph of alginate beads containing adipocytes; 4B: micrograph of alginate beads taken at a magnification of 400X; 4C: micrograph of adipocytes after co-culturing for 2 weeks; 4D: micrograph of mature adipocytes stained with Oil Red O, after co-culturing for one month.

### Example 3: Co-transplantation of immature adipocytes with scaffold

A fibrinogen solution and a thrombin solution were prepared 10 minutes prior to use, as follows: an aprotinin solution was placed in a vial containing lyophilized fibrinogen, which was then allowed to stand for 1 to 2 minutes and gently shaken to be completely dissolved, followed by filling it in a syringe. The thrombin solution was prepared by placing a calcium chloride solution in a vial containing thrombin and shaking the vial until the contents were completely dissolved.

The thrombin solution was added to immature adipocytes obtained by removing the supernatant after final centrifugation in Step (1) of Example 1, such that cells were suspended to a concentration of 1 to 5 x 10⁷ cells/mℓ and the resulting suspension was filled in another syringe. The syringe filled with the fibrinogen solution and the syringe filled with the thrombin-cell suspension were respectively connected to the corresponding parts of a dual syringe kit and an 18-gauge needle was mounted into a spray tip at the end of a syringe. Each 100 µℓ of the fibrinogen solution and thrombin-cell suspension was transplanted while being mixed into the hypoderm above the sternal area of nude mice (Crl:Nu/Nu-nuBR) using the dual syringe kit. Animals were reared over the period of 1 day to 12 months after transplantation, and tissues of the transplantation sites were separated via biopsy. Distribution site, size, engraft rate and survival rate of the transplants were measured. In addition, in order to confirm the evidence of immune rejection between the transplant and host, immunostaining, biochemical examination and molecular biological examination were carried out. Detection of the differentiated human adipocytes was confirmed by immunostaining reaction using the human-specific antibody and a PCR technique using Alu sequence which is a human-specific DNA marker.

From the results of tissue examination for the transplantation sites, it was confirmed that the transplanted immature adipocytes engrafted well into the hypodermic tissues of mice and thereby grew to mature adipocytes.

### Industrial Applicability

As discussed hereinbefore, when immature adipocytes having a cell diameter of 20 to 40 µm, differentiated from preadipocytes which were obtained from human adipose tissues, are used in conjunction with a scaffold for autologous or allogeneic transplantation, maturation of adipocytes with a gradual increase of the volume thereof is achieved at the target transplantation site. Therefore, the adipocyte-biodegradable scaffold composition according to the present invention can be utilized as an effective body volume replacement and can treat various disorders due to defects of soft tissues or aesthetic defects in appearance.

Although the preferred embodiments of the present invention have been disclosed for illustrative purposes, those skilled in the art will appreciate that various modifications, additions and substitutions are possible, without departing from the scope and spirit of the invention as disclosed in the accompanying claims.

## Claims

1. An implantable adipocyte composition for replacing a body volume, comprising immature adipocytes having a cell diameter of 20 to 40 µm, which are obtained by differentiation of adipose tissue-derived preadipocytes into adipocytes.

2. The composition according to claim 1, for use in autologous or allogenic transplantation for a mammal.

3. The composition according to claim 2, wherein the mammal is a human.

4. A method for producing an implantable adipocyte composition, comprising:
a) differentiating undifferentiated preadipocytes, isolated from autologous or allogenic adipose tissues, into adipocytes;
b) collecting differentiated immature adipocytes having a cell diameter of 20 to 40 µm; and
c) culturing the collected immature adipocytes with a biodegradable scaffold *in vitro.*

5. The method according to claim 4, further comprising (d) determining binding and maturation of adipocytes within the biodegradable scaffold.

6. An implantable adipocyte composition, obtained according to any one of claim 4 or claim 5, for use in transplanting adipocytes, wherein the composition comprises immature adipocytes in conjunction with the biodegradable scaffold to be transplanted into a body.

7. The implantable adipocyte composition according to claim 6, wherein the adipocytes and biodegradable scaffold are to be transplanted by an injection.

8. The implantable adipocyte composition according to claim 6, wherein the adipocytes and biodegradable scaffold are to be transplanted by a surgical operation.

9. The implantable adipocyte composition according to any one of claims 6-8, wherein the adipocytes and biodegradable scaffold are to be transplanted for a tissue reconstruction or an aesthetic plastic surgery.

10. The implantable adipocyte composition according to any one of claims 6-9, wherein the immature adipocytes are immature human adipocytes.

## Patentansprüche

1. Implantierbare Adipozytenzusammensetzung zum Ersetzen eines Körpervolumens, umfassend unreife Adipozyten mit einem Zelldurchmesser von 20 bis 40 µm, welche durch Differenzierung von Fettgewebe-abgeleiteten Präadipozyten in Adipozyten erhalten werden.

2. Zusammensetzung nach Anspruch 1 zur Verwendung bei autologer oder allogener Transplantation für ein Säugetier.

3. Zusammensetzung nach Anspruch 2, wobei das Säugetier ein Mensch ist.

4. Verfahren zur Herstellung einer implantierbaren Adipozytenzusammensetzung, umfassend:
a) Differenzieren undifferenzierter Präadipozyten, welche aus autologen oder allogenen Fettgeweben isoliert wurden, in Adipozyten,
b) Sammeln von differenzierten unreifen Adipozyten mit einem Zelldurchmesser von 20 bis 40 µm, und
c) Kultivieren der gewonnenen unreifen Adipozyten mit einem biologisch abbaubaren Zellträger in Vitro.

5. Verfahren nach Anspruch 4, ferner umfassend (d) Ermitteln von Bindung und Reifung der Adipozyten innerhalb des biologisch abbaubaren Zellträgers.

6. Implantierbare Adipozytenzusammensetzung, erhalten nach entweder Anspruch 4 oder Anspruch 5, zur Verwendung beim Transplantatieren von Adipozyten, wobei die Zusammensetzung unreife Adipozyten in Verbindung mit dem biologisch abbaubaren Zellträger zum Transplantieren in einen Körper umfasst.

7. Implantierbare Adipozytenzusammensetzung nach Anspruch 6, wobei die Adipozyten und der biologisch abbaubare Zellträger durch eine Injektion transplantiert werden sollen.

8. Implantierbare Adipozytenzusammensetzung nach Anspruch 6, wobei die Adipozyten und der biologisch abbaubare Zellträger durch einen chirurgischen Eingriff transplantiert werden sollen.

9. Implantierbare Adipozytenzusammensetzung nach einem der Ansprüche 6-8, wobei die Adipozyten und der biologisch abbaubare Zellträger bei einer Gewebsrekonstruktion oder einem ästhetisch-plastischen Eingriff transplantiert werden sollen.

10. Implantierbare Adipozytenzusammensetzung, nach einem der Ansprüche 6-9, wobei die unreifen Adipozyten unreife humane Adipozyten sind.

## Revendications

1. Composition d'adipocytes implantable pour remplacer d'un volume corporel, comprenant des adipocytes immatures présentant un diamètre de cellule de 20 à 40 µm obtenues par différenciation de pré-adipocytes dérivés de tissus adipeux en adipocytes.

2. Composition selon la revendication 1, pour utilisation dans la transplantation autologue ou allogénique chez un mammifère.

3. Composition selon la revendication 2, dans laquelle le mammifère est un être humain.

4. Méthode de production d'une composition d'adipocytes implantable, comprenant :
a) la différenciation de pré-adipocytes non différenciés, isolés de tissus adipeux autologues ou allogéniques, en adipocytes;
b) la collection d'adipocytes immatures présentant un diamètre de cellule de 20 à 40 µm; et
c) la culture des adipocytes immatures collectés avec un échafaudage biodégradable *in vitro.*

5. Méthode selon la revendication 4, comprenant en outre (d) la détermination de la liaison et de la maturation d'adipocytes au sein de l'échafaudage biodégradable.

6. Composition d'adipocytes implantable, obtenue selon l'une quelconque des revendications 4 ou 5, pour utilisation dans la transplantation d'adipocytes, dans laquelle la composition comprend des adipocytes immatures en combinaison avec l'échafaudage biodégradable destiné à être transplanté dans un corps.

7. Composition d'adipocytes implantable selon la revendication 6, dans laquelle les adipocytes et l'échafaudage biodégradable sont destines à être transplantés par une injection.

8. Composition d'adipocytes implantable selon la revendication 6, dans laquelle les adipocytes et l'échafaudage biodégradable sont destines à être transplantés par une opération chirurgicale.

9. Composition d'adipocytes implantable selon l'une quelconque des revendications 6-8, dans laquelle les adipocytes et l'échafaudage biodégradable sont destines à être transplantés pour une reconstruction tissulaire ou une opération de chirurgie plastique esthétique.

10. Composition d'adipocytes implantable selon l'une quelconque des revendications 6-9, dans laquelle les adipocytes immatures sont des adipocytes humains immatures.
